Europäisches Patentamt

European Patent Office

Office européen des brevets

(19)

(11) **EP 0 950 713 A2**

(12) **EUROPÄISCHE PATENTANMELDUNG**

(43) Veröffentlichungstag:
20.10.1999 Patentblatt 1999/42

(51) Int. Cl.⁶: **C12N 15/86**, A61K 48/00

(21) Anmeldenummer: 99102056.1

(22) Anmeldetag: 02.02.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 20.02.1998 DE 19807265

(71) Anmelder: **Centeon Pharma GmbH**
**35002 Marburg (DE)**

(72) Erfinder:
• **Haack, Anja**
**53501 Grafschaft (DE)**
• **Schmitt, Christoph**
**53501 Grafschaft (DE)**

(54) **Rekombinanter adenoviraler Vektor für den Gentransfer**

(57) Es wird ein adenoviraler Transfervektor für den Gentransport einer DNA-Sequenz beschrieben, der aus einem adenoviralen Plasmid hergestellt ist, das keine natürlichen adenoviralen Proteine mehr exprimiert und

a) eine erste DNA-Sequenz mit der linken terminalen invertierten Sequenzwiederholung (ITR) und einem Verpackungssignal des Wildtyp Adenovirus (Serotyp 5),

b) eine zweite DNA-Sequenz mit der rechten terminalen invertierten Sequenzwiederholung (ITR) des Wildtyp Adenovirus (Serotyp 5) sowie

c) Schnittstellen für Restriktionsendonukleasen enthält, die in den zwischen den adenoviralen DNA-Sequenzen einzubauenden Markergenen und/oder therapeutischen Genen nicht vorkommen und bevorzugt

d) die ITRs von Schnittstellen einer Restriktionsendonuklease, die selten (d.h. Erkennungs- oder Schneidesequenzen ≥ 8 Basenpaare), vorzugsweise FseI eingeschlossen sind, wodurch ein Ausschneiden des adenoviralen Anteiles des Transfervektors möglich ist.

EP 0 950 713 A2

## Beschreibung

Gegenstand der Erfindung ist ein für den Gentransport geeigneter adenoviraler Transfervektor sowie ein den Transfervektor enthaltendes Kombinationspräparat, das zur Behandlung der klassischen Hämophilie A eingesetzt werden kann.

Es ist bekannt, dass viele Erkrankungen durch Gendefekte verursacht werden. In diesen Fällen besteht eine Chance für eine dauerhafte Heilung nur dann, wenn es gelingt, das defekte oder fehlende Gen dem Organismus wieder zuzuführen. Deshalb wird verstärkt nach Methoden der somatischen Gentherapie gesucht, die durch eine einzige oder in großen Zeitintervallen wiederholte Verabreichung des intakten Gens zur Gesundung des Patienten führen. Ein Beispiel für diese Bemühungen sind die Versuche, eine Heilung der Hämophilie A durch die somatische Gentherapie zu erreichen.

Die Hämophilie A wird durch einen chromosomalen Defekt hervorgerufen wird, der einen Mangel des zur Blutkoagulation benötigten Faktor VIII zur Folge hat. Der genetische Defekt ist im X-Chromosom lokalisiert und wird durch das weibliche Erbgut übertragen, wobei die Überträgerinnen selbst nicht hämophil sind. Eine erfolgversprechende Behandlung der Hämophilie A ist erst möglich geworden, als Plasmakonzentrate des Faktor VIII zur Verfügung gestellt werden konnten. Bei Faktorenkonzentraten aus humanem Spender-Poolplasma besteht im Prinzip die Möglichkeit, dass bisher nicht bekannte oder nicht sicher identifizierbare infektiöse Agenzien mitübertragen werden, obwohl zwischenzeitlich eingeführte Inaktivierungsverfahren sowie die einzelnen Reinigungsschritte selbst solche Agenzien inaktivieren bzw. entfernen. Die Verwendung von in eukaryotischen Zellen hergestellten rekombinanten humanen Gerinnungsfaktoren reduziert ein etwaiges Risiko ganz erheblich. Nachteilig ist jedoch, dass die Expression des Wildtyp Faktor VIII bisher nur in vergleichsweise geringen Mengen gelingt. Das wird zum Einen damit erklärt, dass das Faktor VIII Molekül ein ungewöhnlich großes Polypeptid mit 2332 Aminosäuren ist, und in den bisher dafür eingesetzten Expressionssystemen die mRNA-Konzentrationen relativ niedrig sind. Des weiteren verläuft der Transport des primären Translationsproduktes vom endoplasmatischen Reticulum zum Golgi-Apparat offensichtlich nicht sehr effizient, was sich aufgrund der niedrigen Sekretionsraten in geringen Ausbeuten im Zellkulturüberstand niederschlägt.

Es hat deshalb bereits Untersuchungen mit dem Ziel gegeben, die cDNA des Faktor VIII zu verkürzen. In den Internationalen Patentanmeldungen WO 86/06101, WO 87/07144 und WO 91/09122 wird bereits über die rekombinante Herstellung von verkürzten Faktor VIII-Derivaten berichtet, die die gleiche biologische Wirkung wie das Wildtyp Faktor VIII-Protein aufweisen, in animalen Zellkulturen aber in erheblich höheren Mengen exprimiert werden.

So haben beispielsweise J.J. Toole et al. über die Herstellung und Expression von Faktor VIII-Derivaten berichtet, denen die Aminosäuren 982 bis 1562 oder die Aminosäuren 760 bis 1639 fehlen (Proc. Natl. Acad. Sci. USA (1986) 83, 5939-5942). D.L. Eaton et al. berichteten über die Herstellung und Expression eines Faktor VIII-Derivats, dem die Aminosäuren 797 bis 1562 fehlen (Biochemistry (1986) 25, 8343 bis 8347). R.J. Kaufman beschrieb die Expression eines Faktor VIII-Derivats, dem die Aminosäuren 741 bis 1646 fehlen (Internationale Patentanmeldung WO 87/04187). N. Sarver et al. berichteten über die Herstellung und Expression eines Faktor VIII-Derivats, dem die Aminosäuren 747 bis 1560 fehlen (DNA (1987) 6, 553 bis 564). M. Pasek berichtete über die Herstellung und Expression von Faktor VIII-Derivaten, denen die Aminosäuren 745 bis 1562 oder die Aminosäuren 741 bis 1648 fehlen (Internationale Patentanmeldung WO 88/00831). K.-D. Langner berichtete über die Herstellung und Expression von Faktor VIII-Derivaten, denen die Aminosäuren 816 bis 1598 oder die Aminosäuren 741 bis 1689 fehlen (Behring Inst. Mitt., (1988) No. 82, 16 bis 25, Europäische Patentanmeldung 0 295 597).

P. Meulien et al. berichteten über die Herstellung und Expression von Faktor VIII-Derivaten, denen die Aminosäuren 868 bis 1562 oder die Aminosäuren 771 bis 1666 fehlten (Protein Engineering (1988), 2 (4), 301 bis 306, Europäische Patentanmeldung 0 303 540). Bei der Expression dieser verkürzten Faktor VIII-Derivate in Säugetierzellinien wurde in der Regel eine zehnmal höhere Faktor VIII Expression beobachtet verglichen mit der Herstellung des Wildtyp Faktor VIII.

Bei der Expression von verkürzten Faktor VIII-Derivaten hat sich immer wieder gezeigt, dass die hochglykosylierte B-Domäne des Faktor VIII-Moleküls zwischen den Aminosäuren Arg-739 und Glu-1649 für die prokoagulatorischeund Cofaktor-Aktivität nicht erforderlich ist. Diese Erkenntnis hat Veranlassung gegeben, nach weiteren verkürzten Faktor VIII-Derivaten zu suchen, die sich in hohen Ausbeuten exprimieren lassen, die volle biologische Wirkung des Faktor VIII zeigen und sich darüber hinaus in geeignete Vektoren einbauen lassen, die eine somatische Gentherapie der Hämophilie A ermöglichen. Die somatische Gentherapie erscheint derzeit als die einzige Behandlungsmethode der Hämophilie A, mit der auf die ständige Gabe von exogen zugeführtem Faktor VIII, der im Organismus des Empfängers nur während einer kurzen Halbwertszeit wirksam bleibt, verzichtet werden kann. Dagegen würde es die somatische Gentherapie ermöglichen, dass durch die einmalige Applikation oder durch wiederholte Applikationen in größeren Zeitabständen (mehrere Wochen bis Jahre) dauerhaft eine ausreichende Bildung von Faktor VIII gesichert wird.

Verschiedene Gentransfersysteme sind in den letzten Jahren entwickelt worden, mit denen sich viele Hoffnungen auf ihre Anwendung zum Zwecke der somatischen Gentherapie verbinden.

Eines der vielversprechendsten Systeme sind die sog. replikationsdefekten, adenoviralen Vektoren, die nicht nur ex vivo, sondern auch in vivo im intakten Gesamtorganismus eine sehr hohe Gentransfereffizienz gewährleisten, wie sie mit keinem anderen derzeit verfügbaren System erreicht werden kann. Ein solches Vektorsystem ist in der Europäischen Patentanmeldung 0 808 905 bereits beschrieben.

In dieses spezielle Vektorsystem kann allerdings weder die Wildtyp Faktor VIII-cDNA noch eine verkürzte Faktor VIII-cDNA, die für ein biologisch aktives Faktor VIII-Derivat kodiert, eingebaut werden, da sie zu groß sind.

Aus der internationale Patentanmeldung WO 96/33280 ist jedoch schon ein adenovirales Helfervirussystem bekannt, das bis zu 36 kB einer heterologen DNA in einen nicht mehr replikationsfähigen adenoviralen Vektor aufnehmen kann. Dieses System besteht aus einem adenoviralen Vektorkonstrukt, einem oder mehreren Helferviren und einer Helferzellinie. Das Vektorkonstrukt kann vervielfältigt und ein Virionpartikel in der Helferzellinie verpackt werden, wenn es zusammen mit einem Helfervirus verabreicht wird, das ein defektes Verpackungssignal enthält. Es stellte sich deshalb die Aufgabe, ein derartiges adenovirales Transfersystem so zu modifizieren und zu verbessern, dass es in der somatischen Gentherapie zum Transfer für therapeutisch einsetzbare Gene verwendet werden kann.

Gegenstand der Erfindung ist deshalb ein adenoviraler Transfervektor für den Gentransport einer DNA-Sequenz, der aus einem adenoviralen Plasmid hergestellt ist, das keine natürlichen adenoviralen Proteine mehr exprimiert und

a) eine erste DNA-Sequenz mit der linken terminalen invertierten Sequenzwiederholung (ITR) und einem Verpackungssignal des Wildtyp Adenovirus (Serotyp 5) und

b) eine zweite DNA-Sequenz mit der rechten terminalen invertierten Sequenzwiederholung (ITR) des Wildtyp Adenovirus (Serotyp 5) sowie

c) Schnittstellen für Restriktionsendonukleasen ent-hält, die in den zwischen den adenoviralen DNA-Sequenzen einzubauenden Markergenen und/oder thera-peutischen Genen nicht vorkommen, wobei bevorzugt

d) die ITRs von Schnittstellen einer Restriktionsendonuklease, die selten (d.h. Erkennungs- oder Schneidesequenzen ≥ 8 Basenpaare), vorzugsweise Fsel eingeschlossen sind, wodurch ein Ausschneiden des adenoviralen Anteils des Transfervektors möglich ist.

Durch die Deletion sämtliche adenoviraler Gene entsteht eine Verpackungskapazität von bis zu 36 kb. Will man ein solches DNA-Fragment, integriert in ein Plasmid aus diesem ausschneiden, so benötigt man flankierende Schnittstellen für Restriktionsendonukleasen, die sehr selten schneiden. Die Erkennungssequenzen dieser Endonukleasen sind meist 4, 6 oder 8 Basenpaare lang. Statistisch gesehen schneidet eine Endonuklease, die eine 6 bp lange Erkennungssequenz erkennt, alle 4096 Basenpaare einmal, bei 8 bp aber nur alle 65536 Basenpaare einmal. Um möglichst vielfältig die Möglichkeiten zu haben, verschiedene DNA-Fragmente in ein solches Mini-Vektor-Konstrukt hineinzuklonieren, ist es sinnvoll, ein sehr selten schneidendes Enzym zu verwenden, um beim Ausschneiden der Mini-Vektor-DNA eingefügte DNA-Fragmente nicht zu zerstückeln.

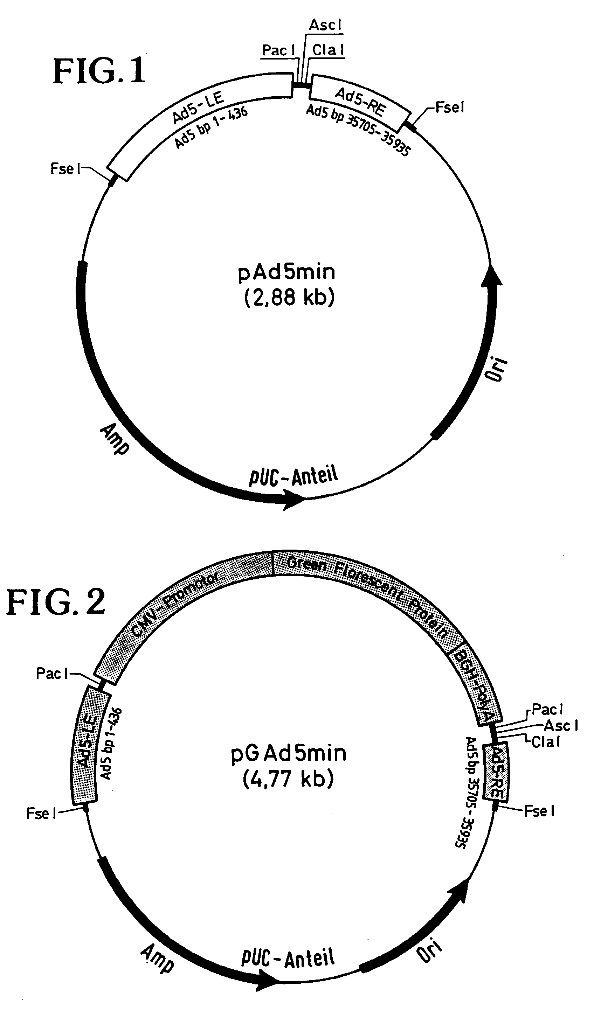

Das in Fig. 1 dargestellt Plasmid pAd5min ist ein Beispiel für einen derartigen adenoviralen Transfervektor. Dargestellt sind die Sequenzen des Plasmids pUC19, an die über die Schnittstelle Fsel zunächst die linke terminale invertierte Sequenzwiederholung und ein Verpackungssignal (ITR und Verpackungssignal= Ad5-LE) mit den Basenpaaren 1 bis mindestens 358 des Wildtyp Adenovirus (Serotyp 5) angefügt ist. Dann folgen die Schnittstellen Pacl, Ascl und Clal, die mit der DNA-Sequenz der rechten terminalen invertierten Sequenzwiederholung ( ITR = Ad5-RE ) bestehend aus den Basenpaaren 35.705 bis 35.935, wenigstens jedoch aus den Basenpaaren 35833 bis 35935 verbunden sind. Anschließend ist erneut eine Fsel-Schnittstelle eingefügt. Dieses Plasmid-Konstrukt erlaubt das Ausschneiden des Ad5-Anteil mittels des Enzyms Fsel.

Die entstandene lineare DNA enthält die gleichen Enden wie der lineare Adenovirus-Wildtyp, verlängert auf jeder Seite um eine Base Cytosin.

Fse I besitzt eine 8 bp lange Erkennungssequenz und schneidet zum Beispiel nicht die Faktor VIII-cDNA. Das Ausschneiden der Mini-Vektor-DNA inklusive Faktor VIII-cDNA und eventueller weiterer Gene/DNA-Sequenzen wird dadurch möglich, ohne die zwischen den beiden begrenzenden Schnittstellen liegenden Sequenzen zu zerschneiden.

Aus demselben Grund wurde auch auf eine gewöhnliche Multiple Cloning Site (MCS) zwischen den adenoviralen Enden verzichtet.

Das Problem einer gewöhnlichen MCS ist, dass viele Schnittstellen der MCS auch wenigstens einmal im Faktor VIII vorkommen. Dadurch ist es nach Einfügen der Faktor VIII-cDNA in die MCS nicht mehr möglich, eine zweite Sequenz in die MCS einzufügen, ohne dabei auch die Faktor VIII-cDNA zu schneiden. Das Einfügen einer zweiten Sequenz zwischen die adenoviralen Enden wäre damit nicht möglich. Die in dem erfindungsgemäßen Plasmid-Konstrukt vorhandene Asc I-Schnittstelle (8 bp Erkennungssequenz) schneidet nicht die Faktor VIII-Sequenz und auch in allen übrigen Sequenzen sehr selten. Fügt man an diese Asc I-Schnittstelle DNA-Fragmente ein, die auf der einen Seite mit Asc I und auf der anderen mit Mlu I geschnitten wurden (Mlu I erzeugt die gleichen überhängenden Enden wie Asc I), so entsteht nach Ligation wieder eine Asc I-Schnittstelle im Mini-Vektor-Konstrukt. Diese kann so mehrmals nacheinander genutzt werden, ohne die Faktor VIII-cDNA oder andere eingefügte Sequenzen zu zerstückeln.

Für die Synthese der Mlu I/Asc I-Fragmente ist deshalb erfindungsgemäß ein spezielles Plasmid konstruiert worden. Dieses besitzt eine Expressionskassette, in die über eine MCS Gene oder cDNAs eingefügt werden können. Die komplette Expressionskassette kann mit Mlu I und Asc I ausgeschnitten und an die Asc I-Stelle im Mini-Vektor-Konstrukt eingefügt werden.

Auch bei Pac I handelt es sich um ein selten schneidendes Enzym (8 bp-Erkennungssequenz), wodurch das Ausschneiden der für in vivo-Versuche nicht gewünschten GFP-Sequenz ohne Zerstückeln der übrigen DNA-Sequenzen möglich wird.

Zusammenfassend ist also festzuhalten, dass der erfindungsgemäße Vektor so konstruiert wurde, dass der Einbau von Markergenen zum Beispiel für das Green Fluorescent Protein, (GFP) oder von therapeutischen Genen wie denen für Faktor VIII oder Faktor IX oder zum Beispiel immunmodulierenden Adenovirus-Genen der E3-Region leicht möglich ist. Die Restriktionsendonukleasen PacI, AscI und FseI besitzen individuelle Schnittstellen, die jeweils 8 bp lang sind und daher nur sehr selten schneiden. Damit erlauben die PacI- und AscI-Schnittstellen das Einfügen unterschiedlichster Gene, da diese nicht von den Restriktionsendonukleasen geschnitten werden. ClaI schneidet zwar häufiger, zeichnet sich aber dadurch aus, dass es im Adenovirus nur einmal schneidet. PacI hat den Vorteil, dass es zur Klonierung des Markergens GFP gut geeignet ist, während AscI und ClaI das wiederholte Aneinanderreihen verschiedener Gene über die gleichen Schnittstellen ermöglichen. Die AscI-Schnittstelle erlaubt die Klonierung eines DNA-Fragments an diese Schnittstelle, wenn das DNA-Fragment in der 5'-Position eine MluI- und in der 3'-Position eine AscI-Schnittstelle besitzt, wobei wiederum eine AscI-Schnittstelle entsteht. Ähnlich kann an die ClaI-Schnittstelle kloniert werden. Das seltene Vorkommen von FseI-Schnittstellen in der DNA ermöglicht das Ausschneiden des Adenovirus-Anteils zusammen mit den eingefügten Genen aus dem Gesamtplasmid.

In einen derartigen adenoviralen Transfervektor können zwischen die beiden adenoviralen Enden Expressionskassetten mit bis zu etwa 35 kb DNA insertiert werden. Damit kann zum Beispiel die komplette oder eine verkürzte cDNA-Sequenz des Faktor VIII und ggf. weitere Expressionskassetten mit immunmodulierenden und/oder DNA-stabilisierenden Genen in das Viruskonstrukt eingebaut werden.

In Fig. 2 ist ein Beispiel für einen erfindungsgemäßen Transfervektor gezeigt, in dem an die PacI-Schnittstelle eine Expressionskassette eingefügt ist, die aus dem Cytomegalovirus (CMV)-Promotor, dem GFP und der Bovine Growth Hormone (BGH)-polyA-DNA besteht. Aus diesem als pGAd5min bezeichneten Plasmidkonstrukt kann aufgrund des seltenen Vorkommens der PacI-Schnittstelle das GFP-Gen zu späteren Zeitpunkten, auch noch nach der Klonierung anderer Gene herausgeschnitten werden.

Für die Herstellung von rekombinanten Viren ist eine gleichzeitige Transfektion mit einer (HEK-293)-Zellinie und mit einem derartigen adenoviralen Transfervektor (zum Beispiel dem pGAd5min) sowie mit einem Helfervirus oder einem Helferplasmid notwendig. Der Transfervektor pAd5min und seine Derivate exprimieren keine adenoviralen Proteine, sofern diese nicht nachträglich insertiert sind. Deshalb müssen die für die Synthese von Adenoviren notwendigen Proteine in trans zur Verfügung gestellt werden. Dafür wird vorzugsweise das Wildtyp-Virus verwendet. Nach Produktion der Viren in Zellkultur müssen sie aufgereinigt und über einen Cäsiumchlorid-Gradienten getrennt werden.

Besonders geeignet ist der vorstehend genannte leere Transfervektor für den Einbau einer cDNA, die für ein Polypeptid mit den Aminosäuren 1 bis 852 und 1.524 bis 2.332 des humanen Faktor VIII kodiert.

Die zur Herstellung dieses Faktor VIII-Peptids erforderliche cDNA-Sequenz wurde aus der cDNA des kompletten Faktor VIII (ATCC 39812 - pSP64-VIII) durch Klonierung in pBluescript II (KS)-Phagemid (Stratagene) unter Verwendung des Restriktionsenzyms Sal I gewonnen. Das dabei entstehende pKS-FVIII wurde mit dem Restriktionsenzym Eco NI (New England Biolabs) gespalten und dabei der für die Aminosäuren 853 bis 1.523 kodierende DNA-Abschnitt entfernt. Anschließend wurden die nicht komplementären DNA-Enden mit der im Beispiel 1 beschriebenen Oligonukleotid-Linker Sequenz verknüpft. Der korrekte Einbau des Linker-Fragments wurde über Sequenzierung verifiziert. Die so hergestellte cDNA für den verkürzten Faktor VIII wurde anschließend in den Expressionsplasmiden pCl-neo(Promega) unter Verwendung von Sall Restriktionsstellen eingefügt. Die Expression erfolgte in Chinese Hamster Ovary (CHO), Monkey Kidney (COS) und Human Embryonal Kidney (HEK-293)-Zellinien.

Zum Vergleich wurde auch die cDNA des Wildtyp Faktor VIII-Gens in den gleichen Expressionssystemen eingesetzt.

Für das verkürzte Faktor VIII-Polypeptid konnte nachgewiesen werden, dass es die gleiche biologische Aktivität aufweist wie der Wildtyp Faktor VIII. Dabei wurden unterschiedlich hohe Expressionsraten in den verschiedenen Expressionsmedien beobachtet, die allerdings in den meisten Fällen erheblich höher lagen als die Expressionsrate des Wildtyp Faktor VIII. Außerdem konnte gezeigt werden, dass das verkürzte Faktor VIII-Polypeptid eine erhöhte Stabilität aufweist und sich im Expressionsmedium anreichern läßt, was auf eine erheblich geringere Empfindlichkeit des verkürzten Faktor VIII gegenüber proteolytischem Abbau hinweist. Dies ist ein deutlicher Unterschied zu dem Wildtyp Faktor VIII, bei dem im Expressionsmedium durch proteolytischen Abbau erhebliche Verluste beobachtet werden. Die Entfernung der B-Domäne des Faktor VIII-Moleküls, deren biologische Funktion bisher nicht geklärt werden konnte, erhöht also nicht nur die Ausbeute, sondern scheint auch die Stabilität des verkürzten Faktor VIII-Derivats gegenüber Proteasen zu verbessern.

Die vorstehend genannten Eigenschaften des verkürzten Faktor VIII-Polypeptids sind empfehlenswerte Voraussetzungen für den Einbau der kodierenden cDNA in einen erfindungsgemäßen Transfervektor, der zu somatischen Gentherapie eingesetzt werden kann. Dabei ist anzustreben, die cDNA für den modifizierten Faktor VIII in die Leber, den physiologischen Syntheseort des Faktor VIII, einzubringen. Aus der Veröffentlichung in J. Biol. Chem. 265, Seite 7318 ff (1990) ist die Expression eines funktionsfähigen Faktor VIII in Fibrobla- sten der menschlichen Haut schon bekannt, wobei der Gentransfer mit einem retroviralen Vektorsystem durchgeführt wurde. Zur Einbringung des Faktor VIII-Gens in die Leber ist dieses Vektorsystem jedoch nicht geeignet. Dagegen ist der oben erwähnte adenovirale Vektor für den Gentransfer in die Leber ideal. Durch die Verwendung eines auf diese Weise modulierten Adenovirus und die Verwendung dieses Vektors zum Gentransfer unter gleichzeitiger transienter Anti-CD4-Behandlung des Empfängerorganismus könnte die Expression der Faktor VIII-cDNA auf einem hohen Niveau über einen langen Zeitraum stabilisiert werden. Die Anti-CD4-Behandlung wird vorzugsweise mit geeigneten monoklonalen Antikörpern gegen CD4-Antigene durchgeführt, die sich zeitlich mit der Applikation des adenoviralen Vektors für den Gentransfer überlappt. Das wesentliche Element dieses Transfersystems ist die Kombination des E3-positiven Vektors mit der Anti-CD4-Strategie zur Verbesserung des hepatischen Gentransfers.

Geeignete monoklonale Anti-CD4-Antikörper sind solche, die die Signaltransduktion vom CD4-Rezeptor blockieren oder den Zielorganismus von CD4-positiven Lymphozyten depletieren. Besonders bevorzugt sind jeweils entsprechende humanisierte monoklonale Antikörper. Dieses adenovirale Vektorsystem ermöglicht es, die erfindungsgemäße cDNA in die Leber als Zielorgan zu transferieren und in Kombination mit transienter Anti-CD4-Behandlung eine erheblich verbesserte Toleranz zu gewährleisten.

Die Erfindung wird durch die folgenden Ausführungsbeispiele näher erläutert:

**Beispiel 1**

**Herstellung einer verkürzten cDNA für den Faktor VIII**

Die aus dem Stamm ATCC 39812 - pSP64 - VIII gewonnene cDNA für den Faktor VIII wurde in pBluescript II (KS)-Phagemid (Stratagene) unter Verwendung der Sall Restriktionsstellen kloniert. Das dabei entstehende Plasmid PKS-FVIII wurde mit Eco NI (New England Biolabs) geschnitten und die nicht komplementären Enden durch Einfügung von elf Basenpaare umfassenden Oligonukleotid-Bindestücken miteinander verbunden. Hierfür wurden folgende Oligonukleotide verwendet:

SEQ ID No. 1: $^{5'}$ G TCA GGC CTC C $^{3'}$
SEQ ID No. 2: $^{5'}$ AG GAG GCC TGA $^{3'}$

Der korrekte Einbau der Verbindungsstücke wurde durch eine automatische Fluoreszenz-Sequenzanalyse nachgewiesen (Applied Biosystems 373 A). Die entstandene verkürzte cDNA (5,2 kb) kodiert für ein verkürztes Faktor VIII-Protein, in dem die Aminosäure 852 mit der Aminosäure 1524 verbunden ist.

**Beispiel 2**

**Expressionssysteme**

Die Wildtyp und die verkürzte Faktor VIII-cDNA wurden in das Expressionsplasmid pCI-neo (Promega) unter Verwendung der Sall-Restriktionsstellen kloniert. Für die Expression wurden CHO-, COS- und HEK-293-Zellinien verwendet. Das Zellmedium bestand aus 10% fetalem Kälberserum (BioWhitaker), l% Penicillin-Streptomycinlösung (BioWhitaker) und Hams F12 (Gibco) für CHO-Zellen oder Dulbeccos Modified Eagle Medium (Gibco) für HEK-293- und COS-Zellinien.

Die Transfektion wurde auf sechs Mikrotiterplatten (Nunc) bei einer Zelldichte von etwa 70% durchgeführt. In jeder Vertiefung der Mikrotiterplatte wurden 15 l Lipofectamin-Reagenz (Gibco), 1,5 g endotoxinfreie Plasmid DNA (gereinigt mit dem Qiagen Plasmid Kit, Endo free maxi) und 1 ml serum-reduziertes Medium (OptiMEM, Gibco) gemischt. Nach einer Inkubation von 30 Minuten wurde die Transfektionsmischung zu den Zellen gegeben und dann bis zu 24 Stunden inkubiert. Die Transfektion wurde dann beendet, indem die Mischung gesammelt und die Zellen sofort mit Normalmedium inkubiert wurden.

**Beispiel 3**

**Antigenbestimmung des Faktor VIII**

Die Antigenbestimmung wurde mit einem hochempfindlichen Faktor VIII-ELISA durchgeführt. Bei diesem Sandwich-ELISA wurden die Platten mit einem monoklonalen anti-human-Faktor VIII-Antikörper (ESH5, American Diagnostica) beschichtet. Der gebundene Faktor VIII wurde mit einem polyklonalen Schaf--anti-human-Faktor VIII-Antikörper (Cedarlane) und einem polyklonalen, Peroxidase-gekoppelten, anti-Schaf IgG-Antikörper vom Esel (Jackson ImmunoResearch Laboratories) nachgewiesen. Gesammeltes Plasma von normalen Individuen wurde als Standard benutzt.

Die Faktor VIII-Aktivität wurde durch einen chromogenen Assay (DADE® Faktor VIII-Chromogen, Baxter) gemessen und sowohl ein einstufiger Assay basierend auf natürlichem, Faktor VIII-freien Plasma als auch ein einstufiger Assay, basierend auf einem durch Immunadsorption Faktor VIII-freien Plasma (Immuno) eingesetzt.

**Beispiel 4**

**Konstruktion eines komplett deletierten adenoviralen Transfervektors**

231 bp des 3'-Endes, bezogen auf den I-Strang des Wildtyp-Adenovirus (Serotyp 5) wurden durch PCR vervielfältigt, wobei die Primer Ad5-RE-A und Ad5-RE-B mit folgenden Basensequenzen eingesetzt wurden:

Basensequenz des Primers Ad5-RE-A (=SEQ ID No. 3):

5' TTG GCG CGC CAT CGA TGC CCA GAA ACG AAA GCC AAA AAA CCC 3'

Basensequenz des Primers Ad5-RE-B (=SEQ ID No. 4):

5' CCC AAG CTT GGC CGG CCA TCA TCA ATA ATA TAC CTT ATT TTG G 3'

Dadurch wurde an das PCR-Produkt in der 5'-Position eine AscI- und eine ClaI-Restriktionsendonukleasen-Schnittstelle und in der 3'-Position eine FseI- und eine Hind III-Schnittstelle angehängt. Über die AscI- und die Hind III-Schnittstellen konnte das PCR-Produkt in das Plasmid pNEB193 (New England Biolabs) kloniert werden. Das entstandene Plasmid wurde pUCRE genannt.

Analog wurden 436 bp des Adenovirus 5'-Endes mit den Primern Ad5-LE-A2 und Ad5-LE-B2 amplifiziert.

Basensequenz des Primers Ad5-LE-A2 (=SEQ ID No. 5):

5' GGG ACG TCG GCC GGC CAT CAT CAA TAA TAT ACC TTA TTT TGG 3'

Basensequenz des Primers Ad5-LE-B2 (=SEQ ID No. 6):

5' TTG ACG TCG GCG CGC CTT AAT TAA CGC CAA CTT TGA CCC GGA ACG C 3'

Mittels der angehängten Schnittstellen für die Enzyme AatII und FseI am 5'-Ende, sowie PacI und AscI am 3'-Ende konnte dieses PCR-Produkt in pUCRE über AatII und AscI kloniert werden.

Dabei entstand das in Fig. 1 dargestellte Plasmid. Dieses Plasmid-Konstrukt erlaubt das Ausschneiden des Ad5-Anteils mittels des Enzyms FseI. Die entstandene lineare DNA enthält die gleichen Enden wie der lineare Adenovirus-Wildtyp, verlängert auf jeder Seite um eine Base Cytosin.

**Beispiel 5**

**Einbau einer Expressionskassette in das Plasmid pAd5min**

Die DNA für das Green Fluorescent Protein (GFP) wurde aus pEGFP-N1 (Clontech) über BamHI- und XbaI-Schnittstellen in pABS.4 (Microbix) kloniert. Der Promotor und das polyA-Signal wurden aus pRc/CMV (Invitrogen) über die Schnittstellen ApoI/EcoRI und KpnI bzw. XbaI und XhoI/SalI in das obige Konstrukt kloniert. Das Plasmid wurde pAGFP/CB genannt. GFP samt Expressionskassette und Kanamycin-Resistenz konnten aus diesem Plasmid mittels der PacI-Schnittstellen isoliert und in pAd5min kloniert werden. Anschließend konnte die Resistenz über SwaI-Schnitt-

stellen aus dem Plasmid entfernt werden. Dieses Plasmidkonstrukt wurde pGAd5min genannt. Aufgrund des seltenen Vorkommens der PacI-Schnittstelle kann das GFP-Gen zu späteren Zeitpunkten und nach Klonierung anderer Gene immer aus dem Plasmid herausgeschnitten werden.

## Sequenzprotokoll:

(I) Allgemeine Angaben

(i) Anmelder:

(A) Centeon Pharma GmbH

(B) Emil-von-Behring-Straße 76

(C) Marburg

(D) Hessen

(E) Bundesrepublik Deutschland

(F) 39041

(ii) Bezeichnung der Erfindung:

Adenoviraler Transfervektor für den Gentransport einer DNA-Sequenz

(iii) Anzahl der Sequenzen:

2

(iv) Zustellanschrift:

(A) Centeon Pharma GmbH

(B) P.O.Box 1230

(C) Marburg

(D) Hessen

(E) Bundesrepublik Deutschland

(F) 35002

(v) Computerlesbare Fassung:

(A) Datenträger:

Diskette

(B) Computer:

IBM PC compatibel

(C) Operating System:

PC-DOS/MS-DOS

(D) Software:

Microsoft Word 6.0

(iv) Patentanmeldungsdaten:

(A) Anmeldenummer:

(B) Anmeldedatum:

(vii) Prioritätsanmeldedaten:

(A) Anmeldenummer:          DE 19807265.1

(B) Anmeldedatum:          20.02.1998

(ix) Telecommunication Information:

(A) Telephon:          06421/39-2069

(B) Telefax:          06421/39-4558

**(2) Angaben zur SEQ ID NO. 1:**

(i) Sequenzcharakter:

(A) Länge:          11 Basenpaare

(B) Art:          Oligonucleotid

(C) Strangform:          Einzelstrang

(D) Topologie:          linear

(vi) Herkunft:          Chemische Synthese

(C) Merkmal:          Linker

(xi) Sequenzbeschreibung SEQ ID NO. 1:

G TCA GGC CTC C          11

**(2) Angaben zur SEQ ID NO. 2:**

(A) Länge :             11 Basenpaare

(B) Art :                Oligonucleotid

(C) Strangform :       Einzelstrang

(D) Topologie :         linear

(vi) Herkunft :         Chemische Synthese

(C) Merkmal :         Linker

(xi) Sequenzbeschreibung SEQ ID NO. 2:

AG GAG GCC TGA            11

**(2) Angaben zur SEQ ID NO. 3:**

(A) Länge :             42 Basenpaare

(B) Art :                Oligonucleotid

(C) Strangform :       Einzelstrang

(D) Topologie :         linear

(vi) Herkunft :         Chemische Synthese

(C) Merkmal :         Primer

(xi) Sequenzbeschreibung SEQ ID NO. 3:

TTG GCG CGC CAT CGA TGC CCA GAA ACG AAA GCC AAA AAA CCC    42

**(2) Angaben zur SEQ ID NO. 4:**

(A) Länge :             43 Basenpaare

(B) Art :                Oligonucleotid

(C) Strangform :        Einzelstrang

(D) Topologie :        linear

(vi) Herkunft :        Chemische Synthese

(C) Merkmal :        Primer

(xi) Sequenzbeschreibung SEQ ID NO. 4:

CCC AAG CTT GGC CGG CCA TCA TCA ATA ATA TAC CTT ATT TTG G   43

**(2) Angaben zur SEQ ID NO. 5:**

(A) Länge :        42 Basenpaare

(B) Art:        Oligonucleotid

(C) Strangform :        Einzelstrang

(D)Topologie :        linear

(vi) Herkunft :        Chemische Synthese

(C) Merkmal :        Primer

(xi) Sequenzbeschreibung SEQ ID NO. 5:

GGG ACG TCG GCC GGC CAT CAT CAA TAA TAT ACC TTA TTT TGG   42

**(2) Angaben zur SEQ ID NO. 6:**

(A) Länge :        46 Basenpaare

(B) Art:        Oligonucleotid

(C) Strangform :        Einzelstrang

(D) Topologie :        linear

(vi) Herkunft :  Chemische Synthese

(C) Merkmal :  Primer

(xi) Sequenzbeschreibung SEQ ID NO. 6:

TTG ACG TCG GCG CGC CTT AAT TAA CGC CAA CTT TGA CCC GGA ACG

C  46

**Patentansprüche**

1. Adenoviraler Transfervektor für den Gentransport einer DNA-Sequenz, **dadurch gekennzeichnet**, dass er aus einem adenoviralen Plasmid hergestellt ist, das keine natürlichen adenoviralen Proteine mehr exprimiert und

    a) eine erste DNA-Sequenz mit der linken terminalen invertierten Sequenzwiederholung (ITR) und einem Verpackungssignal des Wildtyp Adenovirus (Serotyp 5),

    b) eine zweite DNA-Sequenz mit der rechten terminalen invertierten Sequenzwiederholung (ITR) des Wildtyp Adenovirus (Serotyp 5) sowie

    c) Schnittstellen für Restriktionsendonukleasen enthält, die in den zwischen den adenoviralen DNA-Sequenzen einzubauenden Markergenen und/oder therapeutischen Genen nicht vorkommen und vorzugsweise

    d) die ITRs von Schnittstellen einer Restriktionsendonuklease mit einer Erkennungssequenz $\geq$ 8 Basenpaare, vorzugsweise Fsel eingeschlossen sind, wodurch ein Ausschneiden des adenoviralen Anteiles des Transfervektors möglich ist.

2. Transfervektor nach Anspruch 1, **dadurch gekennzeichnet**, dass die erste DNA-Sequenz die Basenpaare 1 bis mindestens 358 und die zweite DNA die Basenpaare 35705 - 35935, wenigstens jedoch die Basenpaare 35833 bis 35935 des Wildtyp Adenovirus (Serotyp 5) umfasst.

3. Transfervektor nach den Ansprüchen 1 und 2, **dadurch gekennzeichnet**, dass er jeweils eine Schnittstelle für die Restriktionsendonukleasen Clal und/oder Ascl enthält, wodurch das wiederholte Aneinanderreihen gleicher oder verschiedener cDNA-Sequenzen an der gleichen Schnittstelle möglich ist.

4. Transfervektor nach den Ansprüchen 1 bis 3, **dadurch gekennzeichnet**, dass er eine Expressionskassette mit der kompletten oder einer verkürzten, für die Aminosäuren 1-852 und 1524-2332 codierenden cDNA-Sequenz des humanen Faktors VIII und gegebenenfalls weitere Expressionskassetten mit immunmodulierenden und/oder DNA-stabilisierenden Genen enthält.

5. Transfervektor nach den Ansprüchen 1 bis 4, **dadurch gekennzeichnet**, dass er zusätzlich ein oder mehrere Markergene enthält, die zur Entfernung aus dem Vektor von geeigneten Schnittstellen umgeben sind.

6. Transfervektor nach den Ansprüchen 1 bis 5, **dadurch gekennzeichnet**, dass er mit Hilfe eines adenoviralen Helfervirus oder Helferplasmids und einer Helferzellinie konstruiert worden ist.

7. Verwendung eines Transfervektors nach den Ansprüchen 1 bis 6 zur Herstellung eines für die somatische Gentherapie einsetzbaren Arzneimittels.

FIG.1

AscI
PacI / ClaI

Ad5-LE
Ad5 bp 1-436

Ad5-RE
Ad5 bp 35705-35935

FseI

FseI

pAd5min
(2,88 kb)

Ori

Amp

pUC-Anteil

FIG.2

CMV-Promotor

Green Florescent Protein

BGH PolyA

PacI

Ad5-LE
Ad5 bp 1-436

PacI
AscI
ClaI

Ad5 bp 35705-35935

Ad5-RE

FseI

FseI

pG Ad5min
(4,77 kb)

Ori

Amp

pUC-Anteil